# EUROPEAN PATENT APPLICATION

(11) **EP 4 226 947 A1**
(43) Date of publication of application: **16.08.2023**
(21) Application number: 21820101.0
(22) Date of filing: 05.10.2021
(51) Int. Cl.: A61K 47/64

(54) **COVALENT CONJUGATES OF THE SARS-COV-2 RECEPTOR-BINDING DOMAIN AND A CARRIER PROTEIN AND VACCINE COMPOSITIONS THAT CONTAIN THEM**

(30) Priority: 05.10.2020 CU 20200069
(71) Applicant: INSTITUTO FINLAY DE VACUNAS, La Habana 11300 (CU); Centro de Inmunologia Molecular, Playa, La Habana 11300 (CU); Universidad de La Habana, La Habana 10400 (CU)
(72) Inventor: VALDÉS BALBÍN, Yury, La Habana 11300 (CU); SANTANA MEDEROS, Darielys, La Habana 11300 (CU); FERNÁNDEZ CASTILLO, Sonsire, La Habana 11300 (CU); GARCÍA RIVERA, Dagmar, La Habana 17100 (CU); GARCÍA RIVERA, Daniel, La Habana 10400 (CU); GARCÍA RICARDO, Manuel, La Habana 10400 (CU); RODRÍGUEZ NODA, Laura Marta, La Habana 10400 (CU); RAMÍREZ GONZÁLEZ, Ubel Jesús, La Habana 17100 (CU); SÁNCHEZ RAMÍREZ, Belinda, La Habana 11300 (CU); BOGGIANO AYO, Tammy, La Habana 11300 (CU); OJITO MAGAZ, Eduardo, La Habana 11300 (CU); VEREZ BENCOMO, Vicente Guillermo, La Habana 11300 (CU); OLIVA HERNÁNDEZ, Reynaldo, La Habana 11300 (CU)
(74) Representative: V.O.
(86) International application number: PCT/CU2021/050009
(87) International publication number: WO 2022/073528

(57) **Abstract**

This invention pertains to biotechnology, more specifically to the field of human health. This document describes in particular conjugates of the SARS-CoV-2 receptor binding domain covalently conjugated to a carrier protein, the method used to obtain them, and the vaccine compositions that contain them. The vaccine compositions described in this invention are useful for the prevention of the SARS-CoV-2 infection as they induce a strong response of neutralizing antibodies.

## Description

### FIELD OF THE INVENTION

This invention pertains to biotechnology, more specifically to the field of human health. This document describes in particular covalent conjugates of the SARS-CoV-2 receptor-binding domain with a carrier protein, the method used to obtain them, and the vaccine compositions that contain them.

### BACKGROUND OF THE INVENTION

COVID-19 is a very recent disease, discovered in Wuhan, China in December 2019, when serious cases of pneumonia of unknown etiology began to be reported. The disease caused by the SARS-CoV-2 virus is characterized by its fast spreading and the appearance of symptoms such as fever, cough, rhinorrhea, a sore throat and dyspnea in the case of symptomatic patients, who account for less than 50 %. The rest of the people infected with the disease are asymptomatic, which is a key factor in the spreading of the virus and represents an epidemiological challenge in terms of its control (WHO Coronavirus disease (COVID-2019) situation reports at www.who.int/emergencies /diseases/novel-coronavirus-2019/situation-reports. Consulted on 13 August 2020).

Other coronaviruses similar to SARS-CoV-2, known as MERS and SARS, have already caused similar epidemics in previous decades. SARS shows greater homology with SARS-CoV-2, and one of the main similarities between them is that both viruses use the ACE2 protein as a receptor to penetrate human cells. Therefore, in both SARS and SARS-CoV-2, the interaction between the receptor binding domain (RED) of the S1 viral protein and the ACE2 (angiotensin-converting enzyme 2) protein is a decisive factor for humans to get infected with the virus. (Walls A et al (2020) Cell https://doi.org/10.1016/j.cell.2020.02.058). The RBD of the S protein of the SARS-CoV-2 is a fragment of approximately 195 amino acids (sequence 333-257), which contains the receptor binding motive (RBM) and is the region in which the virus interacts with the ACE2 receptor. The RBD contains 4 intramolecular disulfide bridges between cysteines Cys336-Cys361, Cys379-Cys432, Cys391-Cys525, and Cys480-Cys488, which helps create a very compact and stable structure (Lan et al (2020), Nature Vol 581: 215-230.

The RBD is a small molecule, whose molecular mass ranges from 25 to 27 kDa depending on the expression host and the carbohydrates incorporated, mainly linked to asparagines N331 and N343 (Chen WH et al., 2017, Journal of Pharmaceutical Sciences 106: 1961-1970).

Strategies for SARS-Cov-2 vaccines include the inactivated virus, genetic constructions that contain viral genetic material incorporated in an adenovirus or as messenger RNA, and vaccines based on viral protein subunits or fragments expressed in genetically modified hosts. In this case, the molecule preferred is the S protein, also known as Spike protein, or a fragment of its structure, i.e., the RBD. Their main advantage is their safety, as this strategy is closer to that of many vaccines in use, nevertheless, its main challenge is the achievement of an immune response that is sufficient to protect from viral infection.

Until now (21 September 2020), there are 149 SARS-CoV-2 candidate vaccines subject to preclinical evaluation and 38 are in clinical trials. Out of those 38, there are at least 13 vaccine candidates (5 under clinical trial and 8 in preclinical studies) with the RBD as specific antigen (DRAFT landscape of COVID-19 candidate vaccines -21 September 2020).

These vaccines consist in the RBD absorbed on alumina at high concentrations of up to 50 micrograms per dose (World Wide Web at clinicaltrials.gov/ct2/show/NCT04466085?term=NCT04466085&draw=2&ra nk=1 Consulted on 17 August 2020). Other vaccine candidates use the RBD protein comprising amino acid residues 319-545 expressed in baculovirus and insect cells, purified and formulated using aluminum hydroxide as the adjuvant. (Yang J et al (2020) Nature Vol 586: 572-592). The RBD in its monomeric form has also been used on animal experiments absorbed on alumina, demonstrating that it can induce neutralizing antibodies without antibody-dependent enhancement (Zang J. et al (2020) bioRxiv 2020.05.21.107565).

None of the aforementioned technical solutions comes close to this invention. The creators of this invention capitalize on the RBD structure to obtain RBD covalently conjugated to carrier proteins. These conjugates can be obtained from any fragment comprising the 333-527 sequence (SEQ ID NO. 2) or extensions thereof.

Surprisingly, the immune response elicited against these conjugates is stronger than the one elicited by non-conjugated monomer RBD in terms of its kinetics and its virus-neutralizing capacity, measured in a virus neutralization assay using SARS-CoV-2 and an ACE2-receptor binding inhibition assay. These are crucial characteristics amidst the pandemic scenario caused by SARS-CoV-2. The vaccine compositions described in the current invention elicit an anti-RBD IgG antibody response on day 7 after immunization with a polarization of cellular response to a Th1 pattern, characterized by the induction of IFNy; therefore, the immunopathological effects reported for coronavirus vaccines that induce a Th2 pattern are not expected in this case.

One weaknesses of coronavirus post-infection acquired immunity with is that it does not last long. The vaccine compositions described in this invention elicit CD8⁺, CD4⁺ and T cells memory response, in particular, lymphocytes specific to the RBD that produce IFNy.

It is worth remarking than none of the technical solutions or scientific publications prior to this invention describes covalent conjugates of RBD to a carrier protein obtained by chemical methods, nor vaccine compositions based on such conjugates.

### SUMMARY OF THE INVENTION

One embodiment of this invention consists in covalent conjugates comprising the SARS-CoV-2 receptor-binding domain (RBD) and a carrier protein. These conjugates are characterized in particular by the fact that the RBD-carrier protein ratio is preferably within the molar range of 1-8 RBD units per one carrier protein, although conjugates with until 13 RBD units can be also obtained. The carrier protein may be selected from the group that comprises tetanus toxoid, diphtheria toxoid, and diphtheria toxin mutant CRM197. The immune effect achieved through conjugation to tetanus toxoid may also be generated through conjugation to other carrier proteins, so the effect of this invention is not limited to the use of tetanus toxoid.

The RBD antigen used for conjugation may have any of the following amino acid sequences, namely 319-541 (SEQ ID NO:1), 333-527 (SEQ ID NO:2), 328-533 (SEQ ID NO:3). SEQ ID NO:1 in particular may also be found in dimeric form. The RBD antigen may be produced in a host selected from the group comprising: mammalian cells (preferably non-human), insect cells, bacteria and yeasts.

Another embodiment of the invention comprises vaccine compositions to induce a protective immune response against SARS-CoV-2, through covalent conjugates made up of the SARS-CoV-2 receptor-binding domain (RBD) and a carrier protein. These vaccine compositions may also include an adjuvant selected from the group comprising any mineral salt such as aluminum hydroxide, aluminum phosphate and calcium phosphate, among others. The conjugate of said vaccine compositions has an RBD concentration range of 1-30 µg per dose while the adjuvant is within a concentration range of 200-1500 µg per dose. These compositions also contain appropriate pharmaceutical excipients.

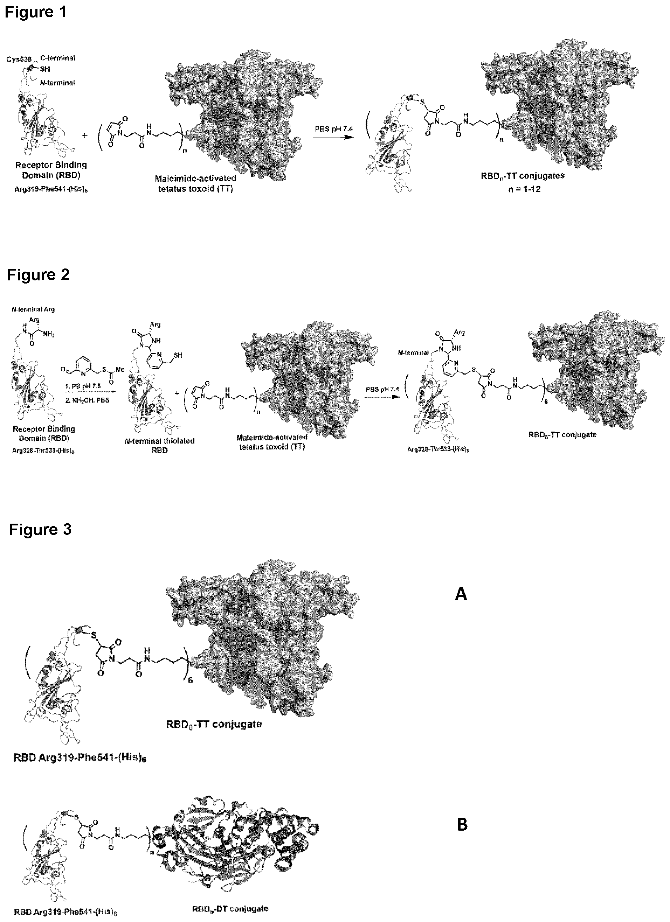

Another embodiment of the invention involves a procedure for obtaining covalent conjugates comprising the following stages: A) Functionalization of the carrier protein for introducing thiophilic groups; B) Covalent conjugation of the carrier protein to the RBD, and C) Purification. This procedure may also include a step consisting in the in situ reduction of the RBD dimer prior to stage A, in which SEQ ID NO: 1 is in its dimeric form (Figure 1). Moreover, the procedure may comprise another step of thiolation of the RBD N-terminus prior to stage A, in which SEQ ID NOs: 1-3 are used (Figure 2). The thiophilic groups introduced in stage A of the procedure are selected from the group comprising maleimides, bromoacetyls, vinylsulphones, acrylates, acrylamides, acrylonitriles, and methacrylates.

Another embodiment of the invention is related to the use of the aforementioned vaccine compositions for the prevention of infection with the SARS-CoV-2 virus. It involves in particular the use of the vaccine compositions described herein when a response of neutralizing antibodies is required.

Finally, there is another embodiment of this invention consisting in the use of the aforementioned vaccine compositions to induce an early IgG antibody response against SARS-CoV-2, applying an intramuscular immunization schedule covering 1 to 3 shots, ranging from 1 to 30 µg of the RBD.

The present invention now provides in a first aspect a covalent conjugate comprising the SARS-CoV-2 receptor-binding domain (RED) and a carrier protein.

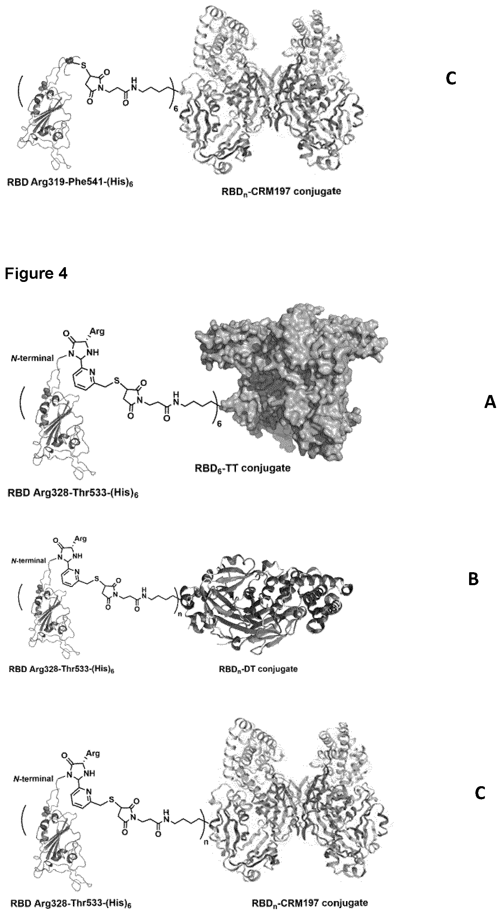

In a preferred embodiment of the covalent conjugate of the invention the carrier protein is selected from the group comprising tetanus toxoid, diphtheria toxoid, and diphtheria toxoid mutant CRM197 (Figure 3 and 4).

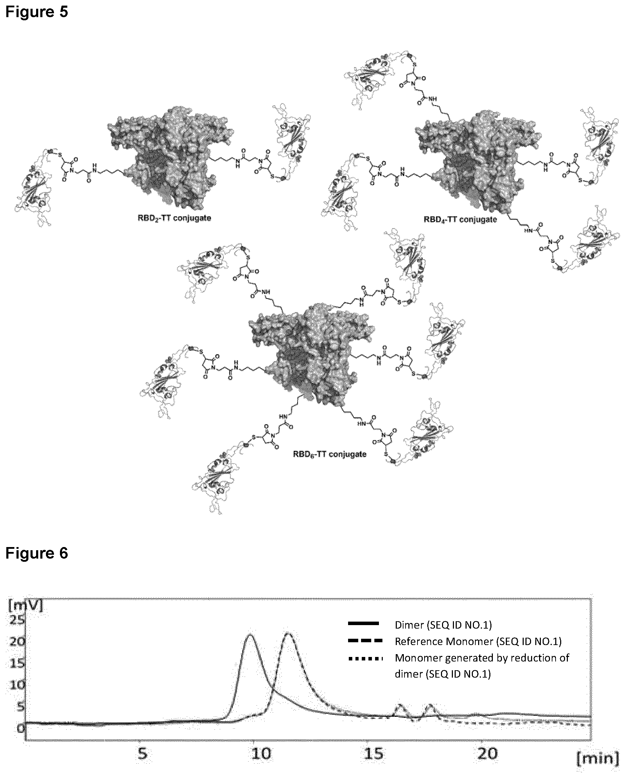

In a preferred embodiment of the covalent conjugate of the invention the RBD-carrier protein molar ratio is within the range of 1 to 8 RBD per carrier protein (Figure 5).

In a preferred embodiment of the covalent conjugate of the invention the RBD is selected from the group comprising SEQ ID NOs: 1, 2 and 3.

In a preferred embodiment of the covalent conjugate of the invention the RBD of SEQ ID NO: 1 is used in its dimeric form.

In a preferred embodiment of the covalent conjugate of the invention the RBD is produced in a host selected from the group comprising or consisting of mammalian cells (preferably non-human), insect cells, bacteria and yeasts.

In another aspect, the present invention provides a vaccine composition comprising the covalent conjugate of the invention as described above. The vaccine composition is preferably used to induce an immune response against SARS-CoV-2.

In a preferred embodiment of the vaccine composition of the invention, the vaccine composition further includes an adjuvant selected from the group comprising or consisting of aluminum hydroxide, aluminum phosphate and calcium phosphate.

In a preferred embodiment of the vaccine composition of the invention, the conjugate is in a concentration range of RBD 1-30 µg per dose.

In a preferred embodiment of the vaccine composition of the invention, the adjuvant is in a concentration range of 200-1500 µg per dose.

In a preferred embodiment of the vaccine composition of the invention, the vaccine composition further includes appropriate pharmaceutical excipients.

In another aspect, the present invention provides a method for the preparation of the covalent conjugate of the invention as described above, the method comprising the steps of: providing a carrier protein and an RBD, wherein the RBD may be in monomeric or in dimeric form, functionalizing the carrier protein by introducing thiophilic groups, covalently conjugating the carrier protein to the RBD, and purifying the conjugate obtained.

In a preferred embodiment of the method for the preparation of the covalent conjugate of the invention wherein a RBD dimer is used, the method comprises an additional step of the reduction of the RBD dimer, preferably *in situ* prior to its conjugation to the carrier protein, preferably prior to functionalization of the carrier protein by introducing thiophilic groups.

In a preferred embodiment of the method for the preparation of the covalent conjugate of the invention, the RBD of any of the SEQ ID NOs: 1-3 may be used, most preferably, the SEQ ID NO:1 is used as the RBD.

In a preferred embodiment of the method for the preparation of the covalent conjugate of the invention, the thiophilic groups introduced are selected from the group comprising or consisting of maleimide, bromoacetyl, vinylsulphone, acrylate, acrylamide, acrylonitrile, and methacrylate.

In another aspect, the present invention provides a conjugate obtained by the method for the preparation of the covalent conjugate of the invention as described above.

In another aspect, the present invention provides the use of the vaccine composition of the invention for the prevention of infection with the SARS-CoV-2 virus, in particular preventing disease caused by SARS-CoV-2 virus infection.

In another aspect, the present invention provides a method of preventing disease caused by SARS-CoV-2 virus infection comprising administering to a subject a therapeutically effective dose of the vaccine composition of the invention as described above.

Preferably, in the use of preventing infection or method of preventing disease according to the invention, the use or method is for the prevention of infection with or disease from the SARS-CoV-2 virus in subjects in need of a neutralizing antibody response after having received two doses or vaccinations of the vaccine composition.

In preferred embodiments of the use and method of preventing infection or disease according to the invention the use or method is to induce a SARS-CoV-2 antibody response by applying an intramuscular vaccination schedule of 1 to 3 doses. Preferably in a scheduled vaccination dose according to the invention, the amount of RBD in a dose is from 1 to 30 µg.

### DESCRIPTION OF DRAWINGS

Figure 1. Scheme of site-selective conjugation of the SARS-Cov-2 Receptor Binding Domain (RED) to tetanus toxoid (TT) activated with thiophilic maleimide groups. The conjugation takes places at the free thiol of Cys538, which is spatially distant from the receptor binding motif (represented in red), and therefore, it does not affect the antigenicity of the RBD.
Figure 2. Scheme of N-terminal selective conjugation of the SARS-Cov-2 Receptor Binding Domain (RED) to tetanus toxoid (TT) activated with thiophilic maleimide groups. The N-terminal thiolation of the RBD takes is carried out with the novel (in-house developed) thioacetyl-pyridinocarbaldehyde reagents that modifies selectively the N-terminal amino group. The N-terminal residue is spatially distant from the receptor binding motif (represented in red), and therefore, it does not affect the antigenicity of the RBD.
Figure 3. Drawing of RBD conjugate based on the RBD (319-541) conjugated at Cys538 using A) Tetanus toxoid, B) Diphtheria toxoid or C) cross-reactive material 197 (CRM197) as carrier protein.
Figure 4. Drawing of RBD conjugate based on the RBD (328-533) conjugated at the N-terminus using A) Tetanus toxoid, B) Diphtheria toxoid or C) cross-reactive material 197 (CRM197) as carrier protein.
Figure 5. Representation RBD-TT conjugates bearing an average of 2, 4 and 6 units of RBD per unit of tetanus toxoid. Conjugates bearing an average of 8, 10 or 13 RBD units were also obtained.
Figure 6. HPSEC Superdex 75 chromatograms, showing the reduction of the RBD's dimer (SEQ ID NO:1) to the RBD monomer, and compared to a reference profile of the RBD monomer.
Figure 7. 10 % SDS-PAGE of the native form of RBD and the RBD derived from the reduction of the RBD dimer (SEQ ID NO:1). R: The sample was incubated in reducing sample buffer before being applied to the gel. NR: The sample was applied in non-reducing sample buffer.
Figure 8. Antigenicity of the monomer obtained by reduction of the RBD dimer (SEQ ID NO:1) with tris(2-carboxyethyl) phosphine (TCEP), as compared to the antigenicity of the native monomer and dimer.
Figure 9. HPSEC Superdex 200 chromatograms of the purified RBD-TT 2 and 3 conjugates (Example 2), as compared to chromatogram of tetanus toxoid.
Figure 10. Recognition of the RBD (319-541)-TT conjugate by the ACE-2 receptor, analyzed by ELISA (A) and by dot blot using anti-RBD polyclonal antibodies (B).
Figure 11. Kinetics of anti-RBD IgG antibodies induced after vaccination on days 0 and 14 with RBD-TT conjugate formulated or not in Al(OH)₃, as compared to RBD formulated in Al(OH)₃. The asterisks indicate significant differences (p≤0.05) between the groups at each time.
Figure 12. Kinetics of anti-TT IgG antibodies induced after vaccination on days 0 and 14 with the RBD-TT conjugate formulated or not in Al(OH)₃.
Figure 13. Inhibition of RBD-ACE2 binding determined in an ELISA with dilutions of antibodies induced on day 28 in mice immunized on days 0 and 14 with the vaccine compositions of the RBD-TT conjugate formulated or not in Al(OH)₃, compared to the RBD formulated in Al(OH)₃.
Figure 14. Anti-SARS-CoV-2 neutralizing activity on day 28 of the sera from the mice immunized with two doses of the RBD-TT conjugate formulated or not in Al(OH)₃, compared to the RBD formulated in Al(OH)₃. The asterisks indicate significant differences (p<0.05).
Figure 15. Production of IFN_{γ}, IL4 and IL17A in spleen cells of mice immunized with RBD-TT formulated in Al(OH)₃ or with the placebo (Al(OH)₃), as determined by a quantitative ELISA. The asterisks indicate significant differences (p<0.05), according to Tukey's test.
Figure 16. Memory cell response induced in mice immunized with two doses of the RBD-TT conjugate formulated in Al(OH)₃. **(A)** CD8⁺ CD44⁺⁺ T lymphocytes , **(B)** IFNy-producing-CD8 T lymphocytes, **(C)** CD4⁺CD44⁺⁺ memory T lymphocytes and **(D)** IFN_{γ}-producing CD4 T lymphocytes. RBD/RBD: Group that received two doses (T0, T14) of RBD-TT and the lymphocytes extracted were stimulated *in vitro* with RBD. Alum/RBD: Group that received two doses (T0, T14) of Alum and the lymphocytes extracted were stimulated *in vitro* with RBD.
Figure 17. HPSEC Superdex 75 Chromatograms, showing the selective thiolation of the N-terminal residue of the RBD monomer (SEQ ID NO:3), compared to the reference profile of the RBD monomer.
Figure 18. **(A)** HPSEC Superdex 75 chromatograms, showing the conjugation of the RBD monomer (SEQ ID NO:3) thiolated at the N-terminal residue (upper panel), the 16 h-reaction mixture of RBD-TT 4 (center panel), and the RBD-TT 4 purified conjugate (lower panel). **(B)** HPSEC Superdex 200 chromatograms of the RBD-TT 4 purified conjugate, compared to tetanus toxoid.
Figure 19. Recognition of the RBD (328-533)-TT conjugate by the ACE-2 receptor, analyzed **(A)** by an ELISA test and **(B)** by anti-RBD polyclonal antibodies, tested by dot blott.
Figure 20. Anti-RED IgG antibodies induced 14 days (T42) after immunization on days 0 and 28 with conjugate vaccine in Phase II clinical trial (19-80 y/o) and Phase I/II clinical trial (3-18 y/o). PCP: Panel of sera from pediatric convalescents.
Figure 21. Anti-RED IgG from saliva samples of vaccinated or unvaccinated subjects

### DETAILED DESCRIPTION OF THE INVENTION

The term "SARS-CoV-2", as used herein, refers to the severe acute respiratory syndrome (SARS) coronavirus 2 (SARS-CoV-2), the causative virus for coronavirus disease 2019 (COVID-19). Testing for positive cases of SARS-CoV-2 can be based on detection of virus RNA sequences by NAAT such as real-time reverse-transcription polymerase chain reaction (rRT-PCR) with confirmation by nucleic acid sequencing when necessary. The viral genes targeted so far include the N, E, S and RdRP genes. The term SARS-CoV-2 disease" and "COVID", are used herein interchangeably, and reference to a viral infectious disease caused by severe acute respiratory syndrome coronavirus-2 (SARS-CoV-2). Common COVID-19 symptoms include fever, cough, and shortness of breath. Muscle pain, sputum production and sore throat are less common. While the majority of cases result in mild symptoms, some progress to severe pneumonia and multi-organ failure. The infection is typically spread from one person to another via respiratory droplets produced during coughing. It may also be spread from touching contaminated surfaces and then touching ones face.

The term "receptor binding domain (RBD)", as used herein refers to the receptor binding domain (RBD) of a coronavirus spike (S) protein of a coronavirus, and includes reference to a part of the coronavirus spike (S) protein that is involved in viral attachment to a receptor on a cell of subject, and subsequent entry into the cell. The cell receptor may be an angiotensin-converting enzyme 2 (ACE2) receptor. Preferably, the RBD comprises or consists of the amino acid sequence of SEQ ID NOs: 1-3. Within the definition of RBD is also foreseen an RBD that is an antigenic part of the amino acid sequence of SEQ ID NOs: 1-3. An RBD as described herein can be modified in that 1-100, preferably 1-50, more preferably 1-10 amino acid residues are added, substituted or deleted from an amino acid sequence of SEQ ID NOs: 1-3 or an antigenic part thereof, preferably wherein said modified RBD binds to a product of an immune response, preferably an antibody, that is elicited when a subject is immunized with a conjugate as described herein, wherein the RBD of said conjugate comprises or consists of an amino acid sequence of SEQ ID NOs:1-3. The term RBD thus includes reference to a sequence having 80%, preferably 90%, more preferably 95% sequence identity with SEQ ID NOs:1-3 and binds to a product of an immune response, preferably an antibody, that is elicited when a subject is immunized with a conjugate as described herein, wherein the RBD of said conjugate comprises or consists of an amino acid sequence of SEQ ID NOs:1-3.

In aspects of this invention the term "a receptor binding domain (RBD) of a coronavirus spike (S) protein" refers only to the RBD portion of the full coronavirus spike (S) protein.

The term "spike (S) protein", or the equivalent term "spike (S) glycoprotein", refers to the coronavirus S protein consisting of a S1 subunit (the N-terminal head) and an S2 subunit (the C-terminal stalk). The S1 subunit mediates virus attachment and entry through its N-terminal S1A domain (comprising sialic acids, a viral attachment factor), and its C-terminal receptor binding domain (RED), which binds to the SARS ACE2 receptor. The S2 subunit is more conserved and mediates viral fusion to the host cell through the fusion peptide (FP) and the two heptad repeats HR1 and HR2. Binding of the SARS-CoV S protein RBD to human ACE2 and CLEC4M/DC-SIGNR receptors results in and internalization of the virus into the endosomes of the host cell.

The term "carrier protein", as used herein, refers to an immunogenic protein to which an antigen such as a protein, oligosaccharide or polysaccharide can be bound. When bound to a carrier, the bound molecule may become more immunogenic. Covalent linking of a molecule to a carrier confers enhanced immunogenicity and T-cell dependence.

As used herein, the term "protein" is used herein to designate a series of amino acid residues, connected to each other by peptide bonds between the alpha-amino and carboxy groups of adjacent residues, also known as "polypeptide". The terms "protein" and "polypeptide"refer to a polymer of amino acids, including modified amino acids (e.g., phosphorylated, glycated, glycosylated, etc.) and amino acid analogs, regardless of its size or function. "Protein" and "polypeptide" are often used in reference to relatively large polypeptides, whereas the term "peptide" is often used in reference to small polypeptides, but usage of these terms in the art overlaps. In the context of the present invention the terms "protein" "polypeptide" and "peptide" are fully interchangeable.

It should be understood that the proteins, including the (RBD) antigens of the invention, may differ from the exact sequences illustrated and described herein. Thus, the invention contemplates deletions, additions and substitutions to the sequences shown, so long as the sequences function in accordance with the methods of the invention. In this regard, particularly preferred substitutions will generally be conservative in nature, i.e., those substitutions that take place within a family of amino acids. For example, amino acids are generally divided into four families: (1) acidic--aspartate and glutamate; (2) basic--lysine, arginine, histidine; (3) non-polar--alanine, valine, leucine, isoleucine, proline, phenylalanine, methionine, tryptophan; and (4) uncharged polar--glycine, asparagine, glutamine, cysteine, serine, threonine, tyrosine. Phenylalanine, tryptophan, and tyrosine are sometimes classified as aromatic amino acids. It is reasonably predictable that an isolated replacement of leucine with isoleucine or valine, or *vice versa;* an aspartate with a glutamate or *vice versa;* a threonine with a serine or *vice versa;* or a similar conservative replacement of an amino acid with a structurally related amino acid, will not have a major effect on the biological activity. Proteins having substantially the same amino acid sequence as the sequences illustrated and described but possessing minor amino acid substitutions that do not substantially affect the immunogenicity of the protein are, therefore, within the scope of the invention. A sequence identity over the whole length of the RBD protein of at least 90%, preferably at least 95%, 96%, 97%, 98%, or 99% is considered to be a functional variant of the RBD protein of SEQ ID NOs: 1-3 and may be used in aspects of this invention. The term "% sequence identity" is defined herein as the percentage of nucleotides in a nucleic acid sequence, or amino acids in an amino acid sequence, that is identical with the nucleotides, resp. amino acids, in a nucleic acid or amino acid sequence of interest, after aligning the sequences and optionally introducing gaps, if necessary, to achieve the maximum percent sequence identity. Methods and computer programs for alignments are well known in the art. Sequence identity is calculated over substantially the whole length, preferably the whole (full) length, of an amino acid sequence of interest. The skilled person understands that consecutive amino acid residues in one amino acid sequence are compared to consecutive amino acid residues in another amino acid sequence.

The term "tetanus toxoid", as used herein, refers to the well-known tetanus toxoid peptide, which peptide represents an epitope of residues 830-844 of tetanus toxin. This sequence has been shown to bind multiple HLA-DR alleles and has been described as universally immunogenic. The amino acid sequence is QYIKANSKFIGITEL.

The term "diphtheria toxoid", as used herein, refers to the inactivated exotoxin secreted by *Corynebacterium diphtheriae,* a single polypeptide chain of 535 amino acids consisting of two subunits linked by disulfide bridges. Diphtheria toxoid is produced worldwide in a standard fashion; in the United States, production and testing procedures are specified in the Code of Federal Regulations.

The term "diphtheria toxoid mutant CRM197", as used herein, refers to a non-toxic mutant of diphtheria toxin, regularly used as a carrier protein for polysaccharides and haptens to make them immunogenic. A single G->A transition in the wild-type diphtheria toxoid sequence leads to the substitution of glycine-52 with glutamic acid (Giannini et al. 1984 Nucleic Acids Res. 12(10): 4063-4069),

The term "covalent", as used herein, refers to a chemical bond in which two atoms share one or more pairs of electrons that hold them together.

The term "covalent conjugate", as used herein, refers to a compound in which an antigen polypeptide is covalently linked to a carrier protein.

The term "covalently conjugating", as used herein, refers to the step of preparing the conjugate through chemical reactions.

The term "vaccine", as used herein, includes reference to a composition of antigenic moieties, usually consisting of modified-live (attenuated) or inactivated infectious agents, or some part of the infectious agents, that is administered, most often with an adjuvant, into the body to produce active immunity. The present invention provides immunogenic compositions comprising a conjugate as described above in a pharmaceutically acceptable carrier. Such a carrier may be an aqueous liquid, or an aerosol composition.

The term "dose", as used herein, refers to a quantity or measured amount of the conjugate or composition of the invention administered or recommended to be administered at a particular time.

The term "adjuvant", as used herein, includes reference to a compound or compounds that, when used in combination with specific vaccine antigens in formulations, augment or otherwise alter or modify the resultant immune responses. Preferably, the adjuvant is an alum (an aluminum salt) such as aluminum hydroxide.

The term "pharmaceutical excipient", as used herein, refers to a material such as an adjuvant, a carrier, pH-adjusting and a buffering agent, a tonicity adjusting agent, a wetting agent, a preservative, and the like. Vaccine excipients are for example described in governmental regulations such as the European Pharmacopoeia and the American 9 CFR, and in handbooks such as: The Handbook of Pharmaceutical Excipients (R. Rowe et al., Pharmaceutical press 2012, ISBN 08571 10276); Remington: the science and practice of pharmacy (2000, Lippincot, USA, ISBN: 683306472).

The term *"in situ* reduction", as used herein, refers to dimer to monomer conversion of RBD as described herein. Reduction of dimers is preferably carried out using tris(2-carboxyethyl) phosphine (TCEP). Preferably, reduction is in situ, meaning that the dimer is admixed with the TCEP and later with carrier protein without any intermediate purification step.

The term "thiolation", as used herein, refers to introduction of a sulfhydryl (SH) group in the RBD N-terminus or N-terminal residue. Thiolation may be achieved by reacting the RBD with an excess of 2-thioacetyl-pyridine-2-carboxaldehyde followed by treatment with hydroxylamine.

The term "functionalizing", as used herein, refers to the introduction of one or more thiophilic groups in the carrier protein. Thiophilic groups may include maleimides, bromoacetyls, vinylsulphones, acrylates, acrylamides, acrylonitriles, and methacrylates. One of skill in the art is well acquainted with methods for introducting one or more thiophilic groups in a protein. Functionalization may for instance be achieved by reacting the carrier protein with maleimido-propionic acid N-hydroxysuccinimide ester in an appropriate buffer.

In aspects of this invention the thiolated RBD is then allowed to react with the thiophilic group(s) functionalized carrier protein to form the conjugate of the invention. Depending on the reaction stoichiometry, the conjugates obtained will contain from one to eight RBD units per unit of the carrier protein. The conjugation procedure described for this invention is chemoselective and residue-specific. A covalent conjugate in accordance with the present invention is preferably not a fusion protein, wherein the RBD and carrier protein are bonded through a peptide bond.

As used herein, the term "administering," refers to the placement of a compound or composition as disclosed herein into a subject by a method or route which results in at least partial delivery of the conjugate at a desired site. Pharmaceutical compositions comprising the compounds disclosed herein can be administered by any appropriate route which results in an effective treatment in the subject. Preferably the administration is by injection, preferably intramuscular.

As used herein, a "subject" means a human or non-human animal. Usually the non-human animal is a vertebrate such as a primate, rodent, domestic animal or game animal. Primates include chimpanzees, cynomologous monkeys, spider monkeys, and macaques, e.g., Rhesus. Rodents include mice, rats, woodchucks, ferrets, rabbits and hamsters. Animals also include armadillos, hedgehogs, and camels, to name a few. Domestic and game animals include cows, horses, pigs, deer, bison, buffalo, feline species, e.g., domestic cat, canine species, e.g., dog, fox, wolf, avian species, e.g., chicken, emu, ostrich, and fish, e.g., trout, catfish and salmon. In some embodiments, the subject is a mammal, e.g., a primate, e.g., a human. The terms, "individual," "patient" and "subject" are used interchangeably herein. Preferably, the subject is a mammal. The mammal can be a human, non-human primate, mouse, rat, dog, cat, horse, cow, or pig, but is not limited to these examples. Mammals other than humans can be advantageously used as subjects that represent animal models of a given condition. A subject is preferably a human. A subject can be male or female. A subject can be one who has been previously diagnosed with or identified as suffering from or having a condition in need of treatment, and optionally, have already undergone treatment. Alternatively, a subject can also be one who has not been previously diagnosed as having a condition. For example, a subject can be one who exhibits one or more risk factors or a subject who does not exhibit risk factors.

A "subject in need" of treatment for a particular condition can be a subject having that condition, diagnosed as having that condition, or at risk of developing that condition.

The term "prevention of infection with the SARS-CoV-2 virus", as used herein, and the term "preventing disease caused by SARS-CoV-2 virus infection", as used herein, refer to preventing or treating COVID-19 disease, or a disease caused by the SARS-CoV-2 virus or variant thereof.

As used herein, the terms "treat," "treatment," "treating," or "amelioration" refer to therapeutic treatments, wherein the object is to reverse, alleviate, ameliorate, inhibit, slow down or stop the progression or severity of a condition associated with a disease or disorder, e.g. cancer or inflammation. The term "treating" includes reducing or alleviating at least one adverse effect or symptom of a condition, disease or disorder. Treatment is generally "effective" if one or more symptoms or clinical markers are reduced. Alternatively, treatment is "effective" if the progression of a disease is reduced or halted. That is, "treatment" includes not just the improvement of symptoms or markers, but also a cessation of, or at least slowing of, progress or worsening of symptoms compared to what would be expected in the absence of treatment. Beneficial or desired clinical results include, but are not limited to, alleviation of one or more symptom(s), diminishment of extent of disease, stabilized (i.e., not worsening) state of disease, delay or slowing of disease progression, amelioration or palliation of the disease state, remission (whether partial or total), and/or decreased mortality, whether detectable or undetectable. The term "treatment" of a disease also includes providing relief from the symptoms or side-effects of the disease (including palliative treatment).

The term "effective amount" as used herein refers to the amount of vaccine antigen needed to alleviate at least one or more symptom of the disease or disorder, and relates to a sufficient amount of pharmacological composition to provide the desired effect. The term "therapeutically effective amount" therefore refers to an amount of vaccine antigen that is sufficient to effect a particular effect when administered to a typical subject. An effective amount as used herein, in various contexts, would also include an amount sufficient to delay the development of a symptom of the disease, alter the course of a symptom disease (for example but not limited to, slowing the progression of a symptom of the disease or its severity), or reverse a symptom of the disease. Thus, it is not generally practicable to specify an exact "effective amount". However, for any given case, an appropriate "effective amount" can be determined by one of ordinary skill in the art using only routine experimentation. An effective amount preferably causes a neutralizing antibody response in the subject.

The term "therapeutically effective amount", as used herein, refers to an amount of a therapeutic agent to treat, ameliorate, counteract, inhibit or prevent a desired disorder or condition, or to exhibit a detectable therapeutic or prophylactic effect. The precise effective amount needed for a subject will depend upon the subject's size and health, the nature and extent of the condition, and the therapeutics or combination of therapeutics selected for administration. The therapeutically effective amount for a given situation can be determined by routine experimentation.

The term "neutralizing antibody response" refers to the generation of antibodies in a subject that bind to and reduce or diminish the activity of a foreign protein, e.g. the RBD of SARS-CoV-2. The activity of the foreign protein can be reduced by a detectable amount, e.g., 10%, 25%, 50%, 75%, or 100% (i.e., completely inactivated), e.g., in comparison to the activity of the foreign protein in the absence of or prior to eliciting the neutralizing antibody response. The activity of the foreign protein will depend on the foreign protein can be determined by any method known in the art.

The term "vaccination", as used herein, refers to the administration of a vaccine to induce a neutralizing antibody response by the recepient's immune system in order to develop protection from a disease.

The term "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

### Method for obtaining the RBD antigen

The coding sequences of the RBD protein are synthesized and subcloned in an appropriate expression vector, preferably pcDNA3.1. The RBD amino acid sequences selected are SEQ ID NOs:1-3 or extensions thereof. The constructions containing target proteins may be expressed in one of the hosts traditionally used in biotechnology, namely mammal cells (CHO, HEK293), insect cells, bacteria and yeasts, preferably CHO cells.

The expressed target protein is harvested by centrifugation and filtration; it is purified on an affinity column, preferably a Ni-Sepharose column, followed by a size-exclusion chromatography purification, preferably in Superdex 200. The purified protein is analyzed by applying appropriate methods such as SDS-PAGE, HPSEC and MS to determine molecular size, purity, identity, and the amino acid sequence, among other molecular characteristics.

To obtain covalent conjugates to carrier proteins the inventors capitalize on the structure of the RBD, which is a globular protein containing 195 amino acids (sequence Thr333-Pro527, SEQ ID NO:2). Said sequence contains four intramolecular disulfide bridges between cysteines (Cys336-Cys361, Cys379-Cys432, Cys391-Cys525, and Cys480-Cys488), creating a very compact and stable structure. This sequence constitutes the biologically relevant structure to be used for conjugation of the RBD as it contains the four immunodominant epitopes described for this molecule, as well as the receptor binding motive. These conjugates can be obtained from any fragment comprising the 333-527 sequence or extensions thereof.

Moreover, the genetic construction used to produce the RBD may include an extension in one or both of the terminal ends of the 333-527 sequence, either in the N-terminus in any of the amino acids from 333 to 300, or in the C-terminus in the 527-560 amino acid region. It is possible to activate one of the terminal ends without affecting the biologically relevant structure by means of the genetic construction itself, specifically by stretching the sequence to include one of the natural amino acids that are considered active, for instance cysteine 538. Another solution consists in introducing active functional groups in one of the terminal ends; an example of this is the thiol group functionalization of the N-terminal amino acid, in example, on arginine 328 of the 328-533 RBD sequence (SEQ ID NO:3).

The amino acid sequence of the target protein from SEQ ID NO:1 contains 9 cysteine, including 8 involved in the 4 intramolecular disulfide bridges (Cys336-Cys361, Cys379-Cys432, Cys391-Cys525, and Cys480-Cys488), which results in a very compact and stable structure. During the purification of the RBD and in the presence of air (mild oxidizing conditions), the RBD of SEQ ID NO:1 may be dimerized by forming a disulfide bridge between two free cysteine at position 538 of two RBD molecules. Nevertheless, if the RBD is kept in an atmosphere of inert gas (i.e. nitrogen or argon), cysteine 538 does not react, the RBD remains in its monomeric form and therefore, it may be involved in chemical reactions with thiophilic groups.

### Method for obtaining covalent conjugates of an RBD to a carrier protein

The carrier protein may be selected from the group comprising tetanus toxoid, diphtheria toxoid, and diphtheria toxin mutant CRM197 or any other protein that performs the same function in vaccines for human use. The methods to obtain and characterize these carrier proteins may be found in the available literature.

The conjugation procedure described for this invention is chemoselective and residue-specific. Said procedure is divided into three stages. The first stage (A) consists in the functionalization of the carrier protein to introduce thiophilic groups, preferably maleimides, although any of the known thiophilic groups may be used, namely bromoacetyls, vinylsulphones, acrylates, acrylamides, acrylonitriles, and methacrylates. Functionalization may be achieved by using maleimido-propionic acid N-hydroxysuccinimide ester in dimethyl sulfoxide (DMSO) at a 100-200 molar ratio to the protein in an appropriate buffer. The functionalized protein is purified by diafiltration using membranes with a MWCO value that suits the protein under purification and the extent of the functionalization is determined by using the modified Ellman procedure.

Stage (B) covers the covalent conjugation of the RBD to the functionalized carrier protein, which are added to a reaction mixture in a mass ratio of 0.2-9.4 RBD:carrier protein (w:w), in an appropriate buffer; the mixture is gently stirred from 4 to 18 h at 5 ± 3°C in an atmosphere of inert gas. The remaining thiophilic groups are blocked by adding the right portion of cystamine.

Stage (C) consists in the purification of the conjugates obtained which can be achieved by diafiltration using a membrane with a MWCO value that suits the carrier protein.

Conjugation may be achieved by using the RBD of SEQ ID NO:1, both in its monomeric and dimeric form. Another step is introduced prior to stage (A) which includes the reduction of the intermolecular disulfide bridge in the RBD dimer accomplished under mild reducing conditions using a reducing agent of disulfide bonds, preferably dithiothreitol (DDT) or tris(2-carboxyethyl) phosphine (TCEP), at concentrations ranging from 125 µM- 420 µM, during 5-20 minutes and at a temperature ranging from 0 to 23°C. These conditions selectively break this bridge in particular without affecting the other four intramolecular disulfide bridges mentioned above. The reduction of the dimer to a monomer may be verified by HPSEC, while the conservation of the four intramolecular disulfide bridges and the antigenicity are respectively checked by SDS-PAGE and an indirect ELISA test.

Another embodiment of this invention comprises another step for the thiolation of the N-terminal residue of the RBD prior to stage A. This procedure entails a reaction with an excess of 2-thioacetyl-pyridine-2-carboxaldehyde (20-50 equivalents in relation to the RBD) for 12-48 hours at 23-37 °C, followed by washings and treatment with an excess of hydroxylamine at ambient temperature during 1-5 hours.

Depending on the reaction stoichiometry, the conjugates obtained will contain from one to eight RBD units per unit of the carrier protein.

### Vaccine compositions and routes of administration

The SARS-CoV-2 vaccines based on the covalent conjugates of the receptor-binding domain to a carrier protein are administered via the intramuscular o subcutaneous route, in RBD doses ranging from 1 to 30 µg, preferably from 5 to 25 µg. These vaccine compositions may contain any mineral salt as an adjuvant, including, but not limited to, aluminum hydroxide, aluminum phosphate and calcium phosphate, in doses ranging from 200 to 1500 µg, preferably from 500 to 1000 µg. Vaccine compositions include appropriate pharmaceutical excipients that can regulate pH, such as phosphate buffers at concentrations ranging from 3.0 to 7.0 mM, isotonic solutions as sodium chloride at concentrations ranging from 50 to 150 mM, and preservatives such as as thiomersal, phenol, 2-phenoxyethanol, methylparahydroxybenzoate, formaldehyde, m-cresol, or other methyl-and propyl-parabens, preferably thiomersal.

These vaccine composition formulations are preferably administered following a schedule of 1 to 3 doses, preferably every 7 to 28 days, more preferably every 14 to 28 days, more preferably every 21 to 28 days. Preferably there is at least a one to three week interval, preferably at least a two week interval, between consecutive doses. A single dose for administration preferably comprises an amount of RBD from 1 to 30 µg. A container, such as an injection flask, may comprise multiple doses for vaccination of multiple subjects. Each dose is preferably about 0.05 to about 1 mL, preferably each dose is about 0.3-0.5 mL, most preferably about 0.3 mL. The dosages may be comprised in the container in concentrated or lyophilized form, and may be diluted or reconstituted with a suitable injection fluid prior to administration.

The administration is preferably intramuscular. The administration is preferably not intravascular, subcutaneous or intradermal. The vaccine compositions proposed in this invention demonstrate their superiority in comparison with a vaccine composition containing the adjuvated, non-conjugated RBD; there are several characteristics of these vaccine compositions which are crucial under the current circumstances of the pandemic, namely, the promptness of the response elicited, and the high levels of antibody titers which act as neutralizers of the SARS-CoV-2 virus and blockers of the RBD-ACE2 receptor interaction. These compositions elicit a Th1 pattern immune response as well as a memory response of CD8⁺, CD4⁺ and T cells specific to the RBD, in particular those producing IFNy.

### Further advantages

The vaccine compositions proposed in this invention demonstrate superior results with clinical trials in the pediatric population.

Furthermore, the vaccine compositions proposed in this invention demonstrate a mucosal IgG response in vaccinated humans. The importance of this is that such an induced mucosal response reduced the transmission of the virus as well as its infectivity. The present inventor consider this a uniquely new property among the presently available Covid-19 vaccines.

It is considered that the presently proposed RBD structure as a part of the complete Spike protein vaccine has important advantages, as the complete Spike protein vaccine shows important adverse event like myocarditis and pericarditis. Those side effects are related with the carboxyl region of Spike protein. This region is not present in the presently proposed RBD-carrier protein conjugate vaccines. It is noteworthy that the presently proposed vaccine composition is very suitable for pediatric use, since the referred side effects of the complete Spike protein vaccines are particularly drastic in pediatric population.

Initial results show that that the immune response of immunosuppressed and immunocompromised subjects is highly beneficial, and better than with other vaccines.

The present inventors have not identified other adverse events, as reported for inactivated viral vaccines or adenovirus vector vaccines, and assessment of the immune response or neutralizing antibody response is higher than for inactivated viral vaccines.

### EXAMPLES

### Example 1. Preparation of RBD (319-541) monomer from the RBD dimerzed at Cys 538.

The RBD dimer (319-541) was dissolved in PBS buffer (35 mM, pH 7.4), 0.5 mM EDTA, to a final protein concentration of 5 to 10 mg/mL. TCEP was added to a final concentration of 125 µM to 420 µM. The reaction was maintained for ten minutes in an argon atmosphere with moderate stirring at ambient temperature.

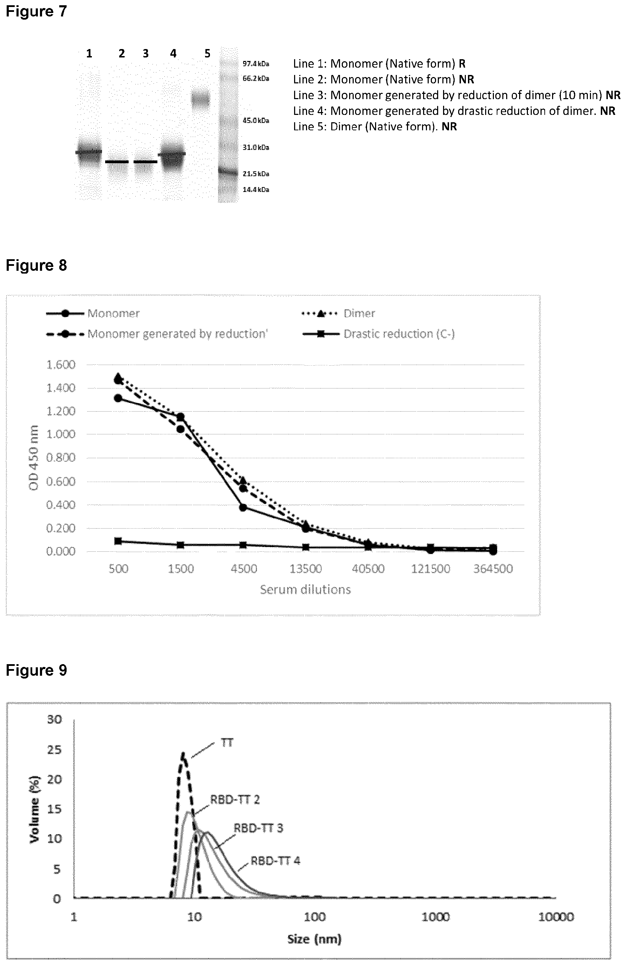

The conversion to the monomeric form and its molecular integrity were verified respectively by HPSEC and polyacrylamide gel electrophoresis. Figure 6 shows the HPSEC profiles revealing that the monomer obtained from reduction has the same molecular distribution as the native monomer. Figure 7 (SDS-PAGE) shows that the RBD monomer regenerated by reduction (line 3) has the same migration pattern as the native RBD monomer (line 2, native conformation), in contrast to a control sample subjected to drastic conditions to reduce inter and intramolecular bridges (line 4), which shows slower migration.

The antigenicity of the monomer obtained by reduction was analyzed by an indirect ELISA using COVID-19 human convalescent serum. Figure 8 proves that the RBD regenerated by reduction was recognized as was the native RBD, while the RBD monomer reduced under drastic conditions, breaking inter and intramolecular disulfide bridges, (Total, C-) is not recognized by the serum.

### Example 2. Preparation of the RBD (319-541)-tetanus toxoid conjugate

Functionalization of the carrier protein: The tetanus toxoid carrier protein (5mg/mL) in HEPES buffer (100 mM, pH 7.8) reacted with maleimido-propionic acid N-hydroxysuccinimide ester in DSMO (75 mg/mL) slowly dropped in the reaction mixture. The reaction was kept for an hour at ambient temperature. The functionalized tetanus toxoid was purified through diafiltration by washing with PBS buffer (35 mM, pH 7.4), 5 mM EDTA. The extent of the functionalization was determined by the modified Ellman procedure.

Conjugation: The functionalized tetanus toxoid was added to a solution of RBD in its monomeric form or to a solution of RBD in situ reduced to the monomer, at an RBD/TT molar ratio of 0.2 to 0.4. The reaction was kept in an argon atmosphere under gently stirring from 4 to 18 h at a 5 ± 3°C. To block the remaining maleimide groups, cysteamine hydrochloride was added at a concentration of up to 157 µM and stirring was maintained for 30 min.

Purification: Purification was achieved by diafiltration using a membrane with a MWCO value of 100 kDa, while washing with PBS (6 mM, pH 7.0).

The RBD/TT ratio was determined by a combination of dot blot densitometry and colorimetry. The content of non-conjugated RBD and the molecular size distribution were determined by HPSEC. Molecular size and polydispersion index were determined by Dynamic Light Scattering (DLS). Table 1 shows that the molar ratio of the conjugates ranges between 1.8 and 6.3 mol RBD/mol TT, while the content of non-bound RBD is lower than 15 %. The molecular size distribution constant (Kd) demonstrates an increase in the molecular size of the conjugates compared to the tetanus toxoid (Kd = 0.31). It is also shown that the relative size of the conjugate population increased as the more RBD moles are incorporated into the carrier protein (Table 1 and Figure 9).

**Table 1. Physicochemical characterization of RBD-TT conjugates.**

| **RBD-TT** | **Conjugat ed RBD monomer** | **RBD/TT molar ratio** | **Kd** | **Molecular size (nm)** | **Polydisper sion index** | **Non-bound RBD** |
|---|---|---|---|---|---|---|
| **RBD-TT 1** | monomer | 1.8 | 0.28 | - | - | 0 % |
| **RBD-TT 2** | reduced monomer* | 2.1 | 0.27 | 13.02 | 0.262 | 0% |
| **RBD-TT 3** | monomer | 6.3 | 0.22 | 23.10 | 0.273 | 10.1 % |
| **RBD-TT 4** | reduced monomer* | 9.5 | 0.19 | 25.32 | 0.294 | 5 % |
| **RBD-TT 5** | reduced monomer* | 13.7 | 0.14 | - | - | 6% |
| **TT** | - | - | 0.31 | 8.75 | 0.192 | - |

| | | | | | | |
|---|---|---|---|---|---|---|
| *Monomer regenerated by the in situ reduction of the RBD dimer | | | | | | |

### Example 3. Demonstration of the recognition of the RBD (319-541)-Tetanus Toxoid conjugate by the ACE 2 receptor and by specific antibodies.

The recognition of the RBD-tetanus toxoid conjugate (RBD-TT) by the ACE2 receptor was analyzed by an ELISA test in which the plate was coated with the recombinant ACE2 receptor. The samples (batches 1 and 2 of RBD-TT, the RBD dimer - as positive control -, and hPDLHys - as negative control) were added at different concentrations (0.001; 0.004; 0.019; 0.078; 0.3125; 1.25 and 5 µg/ml). After incubation, an RBD-specific rabbit polyclonal serum was added, followed by an anti-rabbit IgG conjugated to peroxidase. The reaction was revealed by the corresponding substrate and read at 405 nm.

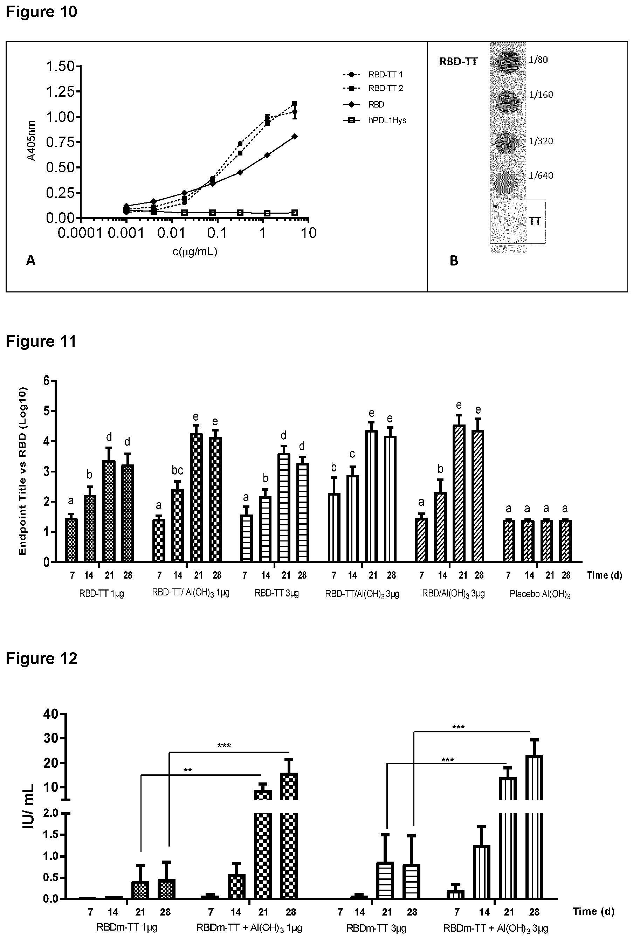

Figure 10A shows that the two conjugate batches are recognized by the ACE2 receptor as well as the RBD dimer (positive control). Therefore, it is proven that in situ reduction and conjugation do not affect the RBD epitopes responsible for the recognition of RBD by the ACE2 receptor.

The antigenicity of the RBD-TT was verified by dot blot using an anti-RBD specific polyclonal IgG serum. Figure 10B shows that the conjugate is strongly recognized by anti-RBD specific antibodies in every dilution tested, while the tetanus toxoid (TT) applied at 1:80 dilution is not recognized. Thus, it is demonstrated that conjugation does not affect the recognition of RBD by the antibodies.

### Example 4. RBD (319-541)-TT conjugate induces a strong antibody response in BALB/c mice.

BALB/c mice were immunized intramuscularly on days 0 and 14, with 0.1 mL of one of the following formulations:
- Group 1: 1 µg of RBD-(319-541)-TT.
- Group 2: 1 µg of RBD-(319-541)-TT absorbed in 500 µg of Al(OH)₃.
- Group 3: 3 µg of RBD-(319-541)-TT.
- Group 4: 3 µg of RBD-(319-541)-TT absorbed in 500 µg of Al(OH)₃.
- Group 5: 3 µg of RBD-(319-541)-TT absorbed in 500 µg of Al(OH)₃.
- Group 6: Placebo. PBS and 500 µg of Al(OH)₃.
Group 6 is the negative control formulation.

Blood was extracted on days 7, 14, 21 and 28. Animal sera were analyzed by an indirect ELISA to determine the anti-RBD antibody titer. The 96-well NUNC Maxisorp microtiter plates were coated with 50 µL of RBD (3 µg/mL) in a carbonate-bicarbonate buffer (pH 9.6) and incubated for one hour at 37°C. Then, the plates were washed three times with a washing solution. Later, the uncoated sites were blocked with 100 µL of 5 % skim milk for 1 hour at 37°C. After washing the plates again as described, the sera (50 µL/well) were added diluted 1:3 in a phosphate buffer (pH 7.2), 1% BSA in serial dilutions, generally starting with a 1:50 dilution . The plates were incubated for 1 hour at 37°C and washed again. Next, 50 µL of a dilution of anti-mouse IgG conjugated to peroxidase was added in a phosphate buffer (pH 7.2), 1% BSA (1:5000) and incubated for one hour. After one last washing, the peroxidase enzyme substrate solution (50 µL/well) was applied. It was then incubated in the dark for 20 minutes and the reaction was stopped with 50 µL/well of 2N H₂SO₄. Absorbance was read at 450 nm in a Multiskan EX ELISA reader (Thermo Scientific). The IgG titer was defined as the inverse of the serum dilution reaching four times the value of the mean absorbance of pre-immune sera (T0) at a 1:50 dilution. For the analysis and presentation of the results, the Log10 of the titer for each animal was calculated. To define respondent animals, a log titer > 1.70 was taken as the cutoff value, corresponding to a > 1:50 serum dilution. A titer value of 25 and a log10 of 1.4 were set for the animals whose titer was lower than the assay detection limit.

Figure 11 shows that the IgG response induced by the immunization with 3 µg of RBD-TT adjuvated in Al(OH)₃ elicits an early antibody response (at days 7 and 14 after immunization), which is significantly stronger (p<0.05) than the one elicited by the same dose of non-conjugated RBD adjuvated in Al(OH)₃. This property is attributable to the conjugation of RBD to a carrier protein. It is also shown that 1 µg of RBD-TT adjuvated in Al(OH)₃ induces an of antibody kinetics similar to that induced by 3 µg of non-conjugated RBD, demonstrating that conjugation enhances the response to the antigen.

It is also seen that adjuvated conjugates increase the anti-RBD antibody level as compared to the same dose of non-adjuvated formulations.

### Example 5. Anti-tetanus toxoid antibody titer induced by the RBD (319-541)-TT conjugate

The sera extracted from mice on days 7, 14, 21 and 28 after immunization according to the procedures described in Example 4 were used to evaluate the induction of anti-TT antibodies. The 96-well NUNC Maxisorp microtiter plates were coated with 100 µL of tetanus toxoid at a concentration of 2.5 µg/mL in PBS (pH 7.2) and incubated overnight at 4°C in a wet chamber. The plates were washed three times with a washing solution. The sera were added (100 µL/well) at various dilutions in a phosphate buffer (pH 7.2), 1% BSA. The plates were incubated for 1 hour at 37°C and washed again. Next, 100 µL of a dilution of anti-mouse IgG conjugated to peroxidase was added in a phosphate buffer (pH 7.2), 1% BSA (1:10000) for one hour at 37°C. After one last washing, 100 µL/well of the peroxidase enzyme substrate solution was applied, incubated in the dark for 25 minutes and the reaction was stopped with 2N H₂SO₄ (50 µL/well). Absorbance was read at 450 nm in a Multiskan EX ELISA reader (Thermo Scientific). The titer was determined by transforming the absorbance values to IU/ml (international units per milliliter); the four-parameter log-logistic function was used to build the reference curve based on serial dilutions of the standard.

Figure 12 shows that the formulations containing the conjugate adjuvated in Al(OH)₃ induced an anti-TT IgG response significantly stronger (p< 0.05) than that elicited by non-adjuvated formulations.

### Example 6. Functional activity of anti-RBD antibodies induced by the RBD (319-541)-Tetanus Toxoid conjugate evaluated through the RBD-ACE2 interaction inhibition assay.

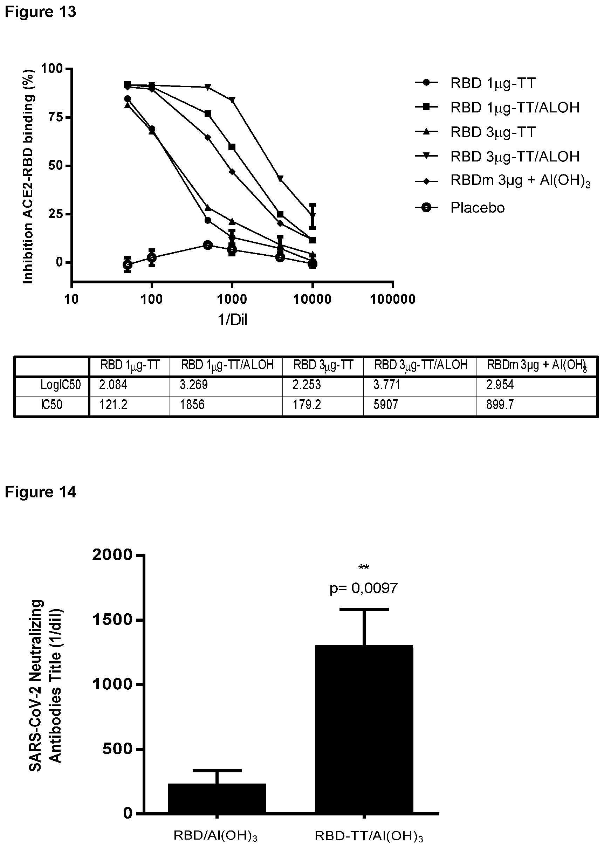

The sera extracted from mice on day 28 after their immunization according to the procedure described in Example 4 were analyzed in an ELISA test to determine their inhibition of the RBD-ACE2 interaction. Another ELISA test was conducted to analyze the inhibition of RBD-ACE2 interaction induced by anti-RBD antibodies. To determine the percentage of inhibition of RBD-ACE2 interaction, the plates coated with mouse ACE2-Fc (5 µg/mL), were blocked; human RBD-Fc that have been incubated for 1 h at 37°C with sera from mice immunized with the various experimental formulations was then added at dilutions ranging from 1:25 to 1:10000. For detection of recognition, an anti-human IgG (gamma chain specific)-alkaline phosphatase conjugate was used diluted in SSTF-T buffer, 0.2% milk. After one last washing, pNPP (1mg/mL, 50 µL/well) was applied in a diethanolamine buffer. The plates were incubated in the dark for 20 minutes and the reaction was stopped with 3M NaOH (50 µL/well). Absorbance was read at 405 nm. The percentage of inhibition was calculated by the following formula: (1-Abs405nm human RBD-Fc + mouse serum/Abs405nm human RBD- Fc) *100. The maximal inhibitory concentration (IC 50) was determined by non-linear regression using the software GraphPad 7.00 (GraphPad Software, Inc., San Diego, CA, USA). Figure 13 shows the inhibition capacity of the serum from the mice immunized with the formulation of the current invention. The antibodies induced by the adjuvated RBD-TT formulations have a greater inhibition capacity than the antibodies induced by non-adjuvated formulations. Moreover, the inhibition capacity (IC 50) of the adjuvated conjugates is 2 (RBD-TT 1 µg /Al(OH)₃) and 6.5 (RBD-TT 3 µg /Al(OH)₃) times higher than that of the non-conjugated formulation (RED 3 µg/Al(OH)₃), which demonstrates that the RBD conjugation enhances the functionality of the antibodies even at lower doses.

### Example 7. Functional activity of anti-RBD antibodies induced by the formulation of the RBD (319-541)-Tetanus Toxoid conjugate evaluated through their SARS-CoV-2 neutralizing capacity.

The SARS-CoV-2 neutralizing capacity of the sera from mice immunized with RBD-TT 3 µg /Al(OH)₃ and RBD 3 µg /Al(OH)₃ at day 28 after the first dose (Example 4) was tested by a colorimetric assay using Neutral Red. The Vero E6 cells were incubated in MEM supplemented with 2 % fetal bovine serum, 25 mM/mL L-glutamine, 2 µg/mL bicarbonate, 80 µg/mL gentamicin, and 5 µg/mL amphotericin B. The supernatant was removed from the plate and PBS, pH 7.2 containing 0.02% Neutral Red was added (100 µl/ well). The plates were incubated for one hour at ambient temperature. The solution was discarded, and the cell monolayers were washed twice with sterile PBS, 0.05% Tween 20. A lysis solution (absolute ethanol: ultrapure water: glacial acetic acid, 50:49:1) was added (100 µL/well). The plate was incubated for 15 min at ambient temperature and measured at 540 nm in a spectrophotometer. The neutralization titer was the highest serum dilution with an optical density value higher than the cutoff value. The cutoff value was the average of the optical density for the wells corresponding to the cell control divided by two.

Figure 14 shows that the neutralizing antibody titers induced by the RBD-TT conjugate are significantly higher ((p< 0.01) than those induced by the non-conjugated RBD, demonstrating the superiority of RBD conjugation as it enhances the functionality of induced antibodies.

### Example 8. Th1 cellular immune response as determined by IFN_{γ} induced by the RBD (319-541)-Tetanus Toxoid conjugate.

The cellular immune response was evaluated in the mice immunized according to the procedure described in Example 4 after extraction of sera on day 21 day after immunization. The splenocytes from the mice immunized either with 1 ug RBD-TT in Al(OH)₃ or with the placebo Al(OH)₃ were isolated and re-stimulated *in vitro* with RBD (5 pg/mL), as determined by a quantitative ELISA. The concentration applied was 1 × 10⁶ cells/mL. IL4, IL17A and IFN_{γ} were determined in the culture supernatant after 72 hours of stimulation.

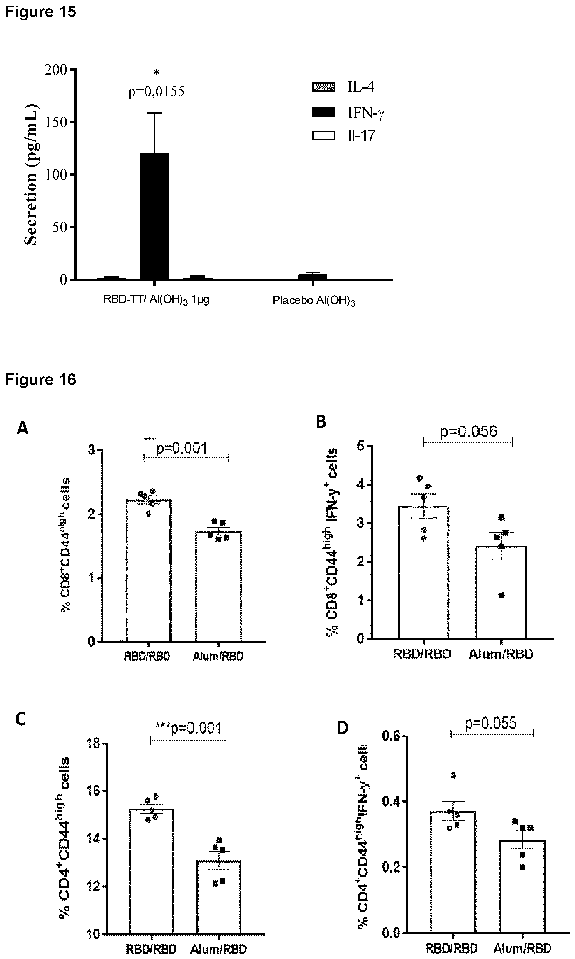

Figure 15 shows that immunization with 1ug of RBD-TT/Al(OH)₃ induces IFNy, demonstrating a Th1 polarization of T cell response. IL4 and IL17A were not detected which evidences that the conjugate does not polarize to a Th2 or Th17 pattern response.

### Example 9. Memory T cells response specific to the SARS-CoV-2 RBD protein, induced by RBD (319-541)-Tetanus Toxoid conjugate.

The memory T cell response was evaluated in mice immunized according to the procedure described in Example 4 on day 21 day after immunization. The splenocytes from the mice immunized either with 1 ug RBD-TT formulated in Al(OH)₃ or with the placebo Al(OH)₃ were isolated, cultured in RPMI 1640, 10 % fetal bovine serum, 100 U penicillin, 100 µg/mL streptomycin, 1mM pyruvate, 50 µM β-mercaptoethanol, and 20 U/mL IL-2 for 72 hours. To activate the cells, 5 µg/ml RBD was added. To block the secretion of cytokines, Brefeldin A (BD Biosciences) was added 4 to 6 hours before staining. The cells were washed with PBS 1X and stained for 30 min at 4 °C with anti-CD8, anti-CD4, anti-CD44 and anti-CD220 (BioLegend). The cells were fixated and permeabilized to facilitate intracellular staining with anti-IFN_{γ} and anti-IL4 (BioLegend). Cytometry data were obtained on a Gallios flow cytometer (Beckman Coulter) and results were processed by the software Kaluza (Beckman Coulter).

Figure 16 shows that immunization with 1 ug of RBD-TT/Al(OH)₃ generates RBD-specific CD4⁺ (C) and CD8⁺ (A) memory T cells, with phenotype CD44⁺⁺. The immunization also increased CD8 (B) and CD4 (D) memory T cells producing IFN_{γ}.

### Example 10. Preparation of the RBD (328-533) thiolated at its N-terminus

RBD (328-533) at a final concentration of 20-200 µM in 5-10 mL PBS (50 mM, pH 7.5, 5 mM EDTA) was treated with 2-thioacetyl-pyridine-2-carboxaldehyde (final concentration 1-10 mM). The reaction mixture was stirred at a temperature of 23-37°C for a time ranging between 12 and 48 h. Purification was achieved through diafiltration by washing with PBS (35 mM, pH 7.4, 5 mM EDTA). The functionalized RBD (328-533) was concentrated up to 20-200 µM and treated with hydroxylamine hydrochloride to a final concentration of 40 mM. The reaction mixture was stirred at ambient temperature in a nitrogen atmosphere for 1 to 5 h and the degree of conversion >90% was determined by the Ellman procedure.

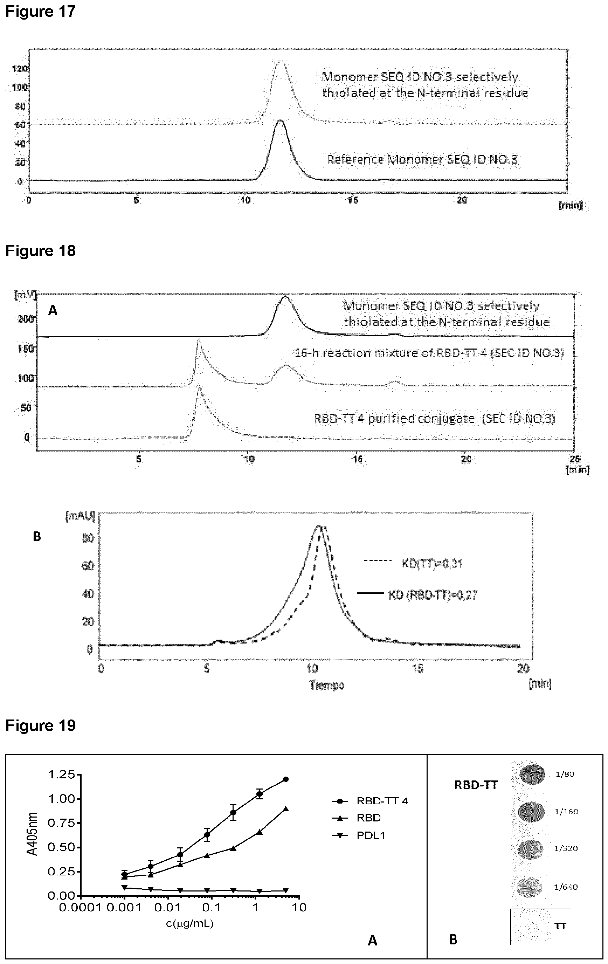

The level of conversion of the N-terminal residue determined by mass spectrometry was higher than 80 %. HPSEC analysis evidenced that no aggregation occurred during the reaction, and that thiolated RBD preserved its molecular size distribution (Figure 17).

### Example 11. Conversion of the N-terminal thiolated RBD (328-533) into the RBD (328-533) tetanus toxoid conjugate.

The thiolated RBD (328-533) is conjugated to the tetanus toxoid following the procedure described in Example 2.

Figure 18 A shows HPSEC Superdex 75 chromatograms for RBD,, the reaction mixture after 16-h of conjugation and the purified conjugate. The purified conjugate has an RBD-TT molar ratio of 1.9, and a non-bound RBD content lower than 15 %. The HPSEC Superdex 200 chromatogram (Figure 18B) shows an increase in the molecular size of the conjugate (Kd=0.27) compared to the carrier protein (Kd=0.31).

The conjugates obtained from SEQ ID NO: 1 and SEQ ID NO:3 following the procedure described in Example 2 have similar physicochemical characteristics.

### Example 12. Preservation of the recognition of the RBD (328-533)-tetanus toxoid conjugate by the ACE2 receptor and by specific RBD antibodies.

The recognition of the RBD-tetanus toxoid conjugate by the ACE2 receptor and by specific anti-RBD antibodies was evaluated as described in Example 3.

Figure 19A shows that the RBD-tetanus toxoid conjugate is recognized by the ACE2 receptor as well as it is recognized in the RBD positive control (RBD in the absence of tetanus toxoid). Therefore, it is proven that neither the thiolation of the N-terminal residue of RBD nor the conjugation processes affect the RBD epitopes responsible for the recognition of RBD by the ACE2 receptor.

The antigenicity of the conjugate was verified by dot blot using an anti-RBD specific polyclonal IgG serum. Figure 19B shows that the conjugate is strongly recognized by the anti-RBD specific antibodies in all dilutions tested, while the tetanus toxoid (TT) at 1:80 dilution is not recognized. Thus, it is demonstrated that conjugation does not affect the recognition of RBD by the antibodies.

### Example 13. RBD (319-541)-TT conjugate elicits strong antibody response in humans, especially in pediatric population.

Vaccine formulation that includes RBD-(319-541)-TT in alum was evaluated in clinical trials in a two doses (T0, T28 days) schedule. Procedures of clinical trials in adult (Phase II, 19-80 years old) and pediatric population (Phase I/II, 3-18 years old) are described in: https://rpcec.sld.cu/trials/RPCEC00000347-En, https://rpcec.sld.cu/trials/RPCEC00000374-En

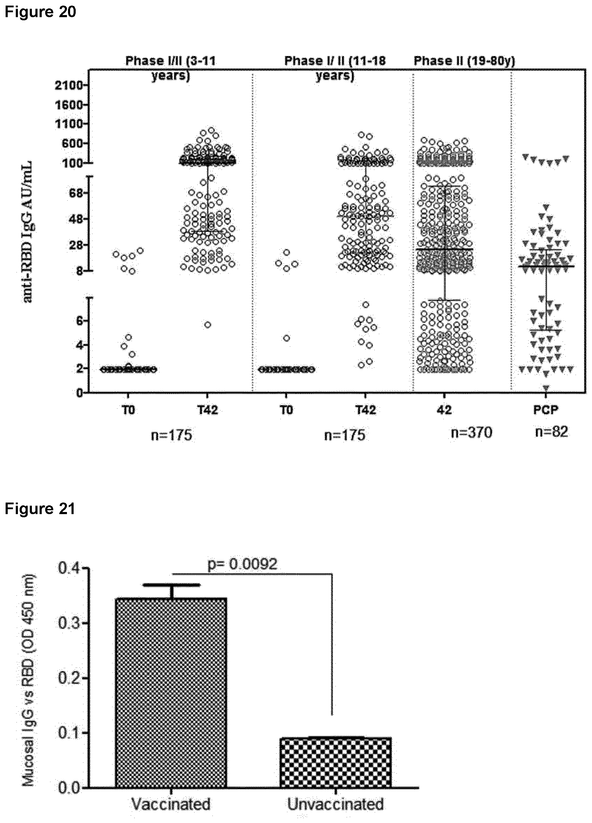

Figure 20 shows results of specific anti-RBD IgG in serum 14 days after second dose in both clinical trials. High levels of antibodies were raised in all age groups with 74% of seroconvertion in adult population (19-80 years old). Notably, children reached 92.8% and 99.3 % of seroconvertion in 12-18 y/o and 3-11 y/o groups, respectively. In addition, medians of both pediatric groups: 50.3 (15.9; 62.0 in 12-18 y/o) and 99.8 (39.1; 216.8 in 3-11 y/o) were superior to the median of a serum panel made with COVID-19 convalescent children: 8.7 (3.4; 15.7).

### Example 14. RBD (319-541)-TT conjugate induces specific mucosal IgG in humans

Saliva from subjects immunized with two doses (T0, 28 days) of a formulation of RBD-(319-541)-TT in alum and a booster dose of dimeric RBD in alum (T56) as described: https://rpcec.sld.cu/trials/RPCEC00000360-En was analyzed by *"in* house" ELISA assay. RBD as coating (5 µg/mL) and PBS-BSA 3 % as blocking. Saliva samples were evaluated pure by duplicated. After incubations steps, peroxidase anti-IgG human conjugate (Sigma A6029, 1:2500) in appropriate buffer were added. The final fluorimetric reaction was induced by adding OPD substrate. The results was expressed in absorbance values.

Figure 16 shows that subjects that were immunized elicited a specific anti-RBD IgG response in saliva.

## Claims

1. A covalent conjugate comprising the SARS-CoV-2 receptor-binding domain (RBD) and a carrier protein.

2. The covalent conjugate of claim 1, wherein the carrier protein is selected from the group comprising tetanus toxoid, diphtheria toxoid, and diphtheria toxoid mutant CRM197.

3. The covalent conjugate according to any of the claims 1 to2, wherein the RBD-carrier protein molar ratio is within the range of 1 to 8 RBD per carrier protein.

4. The covalent conjugate according to any of the claims 1 to 3, wherein the RBD is selected from the group comprising SEQ ID NOs: 1, 2 and 3.

5. The covalent conjugate according to any of claims 1 to 4, wherein the RBD of SEQ ID NO: 1 is used in its dimeric form.

6. The covalent conjugate according to any of claims 1 to 5, wherein the RBD is produced in a host selected from the group comprising: mammal cells, insect cells, bacteria and yeasts.

7. A vaccine composition used to induce an immune response against SARS-CoV-2 which is **characterized by** comprising the covalent conjugate of any of claims 1 to 5.

8. The vaccine composition of claim 7, which also includes an adjuvant selected from the group comprising: aluminum hydroxide, aluminum phosphate and calcium phosphate.

9. The vaccine composition of claim 7, wherein the conjugate is in a concentration range of RBD 1-30 µg per dose.

10. The vaccine composition of claim 8, wherein the adjuvant is in a concentration range of 200-1500 µg per dose.

11. The vaccine composition of claims 7 to 10, which also includes appropriate pharmaceutical excipients.

12. A procedure for the preparation of the covalent conjugate of any of claims 1 to 5, consisting in the following stages: A) Functionalization of the carrier protein for introducing thiophilic groups; B) Covalent conjugation of the carrier protein to the RBD, and C) Purification.

13. The procedure of claim 12, which includes an additional step consisting in the in situ reduction of the RBD dimer prior to stage A.

14. The procedure of claim 12, which includes an additional step consisting in the thiolation of the RBD N-terminus prior to stage A.

15. The procedure of claim 13, wherein SEQ ID NO:1 is used.

16. The procedure of claim 14, wherein any of the SEQ ID NOs:1-3 is used.

17. The procedure of claim 12, wherein the thiophilic groups introduced in stage A are selected from the group comprising: maleimide, bromoacetyl, vinylsulphone, acrylate, acrylamide, acrylonitrile, and methacrylate.

18. A conjugate obtained according to the procedure of any of claims 12 to 17.

19. Use of the vaccine composition of any of claims 7 to 11 for the prevention of infection with the SARS-CoV-2 virus.

20. Use of the vaccine composition of any of claims 7 to 11 for the prevention of infection with the SARS-CoV-2 virus when a neutralizing antibody response is required after two doses of the vaccine composition.

21. Use of the vaccine composition of any of claims 7 to 11 to induce a SARS-CoV-2 antibody response applying an intramuscular vaccination schedule of 1 to 3 doses of RBD from 1 to 30 µg.
